# EUROPEAN PATENT APPLICATION

(11) **EP 3 978 234 A1**
(43) Date of publication of application: **06.04.2022**
(21) Application number: 20813137.5
(22) Date of filing: 27.05.2020
(51) Int. Cl.: B32B 5/22, B32B 27/30, B32B 7/05, A61B 5/0408

(54) **NANOMESH MULTILAYER BODY, METHOD FOR PRODUCING CONDUCTIVE CIRCUIT AND NANOMESH BONDING KIT**

(30) Priority: 29.05.2019 JP 2019100247; 20.08.2019 JP 2019150227
(71) Applicant: Mitsubishi Chemical Corporation, Chiyoda-ku Tokyo 100-8251 (JP)
(72) Inventor: TOYOSU, Kentaro, Tokyo 100-8251 (JP); MURASE, Tomohide, Tokyo 100-8251 (JP); ONO, Yuuki, Tokyo 100-8251 (JP); KOIDE, Fumiyo, Tokyo 100-8251 (JP); KOMORI, Takashi, Tokyo 100-8251 (JP)
(74) Representative: Ter Meer Steinmeister & Partner
(86) International application number: PCT/JP2020/020911
(87) International publication number: WO 2020/241685

(57) **Abstract**

The present invention aims to provide a nanomesh laminate with which a conductive nanomesh material can be easily placed on a desired site and which is less likely to undergo distortion upon attachment. The object is achieved with a nanomesh laminate including: a mesh-shaped base material (A); and a nanomesh layer (B) containing a polyvinyl alcohol resin as a main component; the mesh-shaped base material (A) and the nanomesh layer (B) being layered next to each other, preferably achieved with a nanomesh laminate including: a nanomesh layer (B) containing polyvinyl alcohol as a main component; and a conductive substance layer (C); on a mesh-shaped base material (A).

## Description

### TECHNICAL FIELD

The present invention relates to a nanomesh laminate, preferably a conductive nanomesh laminate. The present invention also relates to a method of producing a conductive circuit using the conductive nanomesh laminate, and a nanomesh-attaching kit comprising the nanomesh laminate.

### BACKGROUND ART

Flexible electronics where an electronic device is formed on a flexible base material has been studied, and research on their application to a living body has also proceeded.

For example, as a member which has high surface followingness and which can be stably used for a long period, an electronic functional member comprising: a base material in which an opening section is formed; and a fiber net suspended by the periphery of the opening section, which periphery functions as an outer frame, has been proposed (see Patent Document 1).

Preparation of a skin-attachable nanomesh sensor having both air permeability and elasticity using a polyvinyl alcohol (PVA) resin, which is biocompatible and whose biodegradation can be expected, has also been proposed (see Non-Patent Document 1).

In the nanomesh sensor disclosed in Non-Patent Document 1, one side of a PVA nanofiber mesh prepared by the electrospinning method is coated with a conductive metal or the like. By placing the nanomesh sensor on the skin, and spraying water thereto, the nanofiber mesh of the conductive metal or the like can be allowed to adhere to the surface of the skin. The nanofiber mesh of the conductive metal or the like after the adhesion can follow shape changes and the like of the skin, can be applied to joints and the like, and can be used for, for example, wiring to various sensors. In particular, by using gold as the conductive metal or the like, and since the mesh structure does not inhibit cutaneous respiration, discomfort and inflammation due to attachment of the nanomesh can be remarkably reduced.

### PRIOR ART DOCUMENTS

### [Patent Document]

[Patent Document 1] JP 2016-112246 A

### [Non-Patent Document]

[Non-Patent Document 1] "Successful development of a skin-attachable nanomesh sensor which enables cutaneous respiration", [online], University of Tokyo, Japan Science and Technology Agency, Keio University, RIKEN, [search on February 25, 2019], internet <URL:http://www.jst.go.jp/pr/announce/20170718/>

### SUMMARY OF THE INVENTION

### PROBLEMS TO BE SOLVED BY THE INVENTION

In the electronic functional member of Patent Document 1, a fiber net is formed by the electrospinning method, and the fiber net is coated with a conductive substance by the vapor deposition method, the sputtering method, or the like.

However, for practical use of the nanomesh sensor, there have been problems to be solved in relation to its usability, such as damage of the nanomesh before the use due to fineness of the nanomesh itself, and damage of the nanomesh upon detachment of the nanomesh from the base material.

One solution to such unintended damage may be use of a metal foil as a release material, but it is unavailable because of incompatibility with the electrospinning method. In cases where a resin sheet is used as the release material, there is the problem of detachability. Therefore, a realistic method has been limited to use of a silicone-processed greaseproof paper (cooking sheet) as the release material. However, detachment of the silicone-processed greaseproof paper is still difficult, and the nanomesh after the detachment has a problem concerning its smoothness. It is thus clear that the silicone-processed greaseproof paper has a problem concerning its handling.

Under such circumstances, the present inventors proposed a conductive nanomesh material comprising a nanomesh coated with a conductive substance, the nanomesh being placed on a release material having a particular tape peel force (Japanese Patent Application No. 2019-65956). By detaching the nanomesh from the separating agent, placing the nanomesh on the skin, and then spraying water or the like thereto to dissolve the nanomesh, wiring composed of the conductive substance is formed on the skin.

However, since the nanomesh after detached from the release material is thin and light, it cannot be easily attached to a desired site. For example, in cases where the site where the nanomesh is to be attached is small, positioning of the nanomesh is difficult, while in cases where the site where the nanomesh is to be attached is large, distortion occurs between the portion that has already adhered and the portion that has not yet adhered, leading to difficulty in uniform attachment.

An object of the present invention is to provide a nanomesh laminate which allows simple attachment of a nanomesh material on a desired site and which is less likely to cause distortion upon the attachment.

### MEANS FOR SOLVING THE PROBLEMS

In order to solve the above problem, the present inventors continued improvement to discover that a nanomesh laminate which can be easily attached and which is less likely to cause distortion upon the attachment can be provided with a nanomesh laminate comprising:
a mesh-shaped base material (A); and
a nanomesh layer (B) containing a polyvinyl alcohol (PVA) resin as a main component,
   the mesh-shaped base material (A) and the nanomesh layer (B) being layered next to each other.

Further, the present inventors discovered that, by providing a mesh-shaped base material (A) on the side opposite to the placement side of a laminate of a nanomesh layer (B) containing a PVA resin as a main component and a conductive substance layer (C), positioning of the nanomesh laminate itself can be easily carried out, and distortion between the portion that has already adhered and the portion that has not yet adhered can be reduced.

In the case where the mesh-shaped base material (A) is provided on the side opposite to the placement side of the laminate of the nanomesh layer (B) and the conductive substance layer (C), since the mesh-shaped base material (A) has a mesh shape, water or the like can be supplied to the laminate using a sprayer or a carrier such as absorbent cotton impregnated with water or the like after placement of the nanomesh laminate including the mesh-shaped base material (A) on the desired site of placement. The water or vapor supplied to the laminate, or condensed water or the like on the surface of the adherend dissolves the nanomesh layer (B), which contains a PVA resin as a main component. By this, the conductive substance layer (C) can be attached to the desired site. Further, since the mesh-shaped base material (A) is present as it is upon the attachment, distortion is less likely to occur upon the attachment.

More specifically, the present invention includes the following.
[1] A nanomesh laminate comprising:
   a mesh-shaped base material (A); and
   a nanomesh layer (B) containing a polyvinyl alcohol resin as a main component,
   the mesh-shaped base material (A) and the nanomesh layer (B) being layered next to each other.
[2] The nanomesh laminate according to [1], comprising:
   the nanomesh layer (B) containing a polyvinyl alcohol resin as a main component; and
   a conductive substance layer (C),
   on the mesh-shaped base material (A).
[3] The nanomesh laminate according to [1] or [2], wherein
   the nanomesh layer (B) containing a polyvinyl alcohol resin as a main component,
   the conductive substance layer (C), and
   a protection layer for protecting these,
   are layered in this order on the mesh-shaped base material (A) .
[4] The nanomesh laminate according to any one of [1] to [3], wherein the mesh-shaped base material (A) has an opening area of 55 to 80%.
[5] The nanomesh laminate according to any one of [1] to [3], wherein
   a mesh-shaped base material (A) having an opening area of 55 to 80%, and
   a nanomesh layer (B1) containing polyvinyl alcohol as a main component, and containing a cosmetic component or a pharmaceutical component,
   are layered next to each other.
[6] The nanomesh laminate according to any one of [1] to [3], wherein the mesh-shaped base material (A) has an opening area of 10 to 45%.
[7] The nanomesh laminate according to any one of [1] to [3], wherein
   a mesh-shaped base material (A) having an opening area of 10 to 45%, and
   a nanomesh layer (B1) containing polyvinyl alcohol as a main component, and containing a cosmetic component or a
   pharmaceutical component,
   are layered next to each other.
[8] A method of producing a conductive circuit, comprising the steps of:
   placing the nanomesh laminate according to any one of [1] to [7] on a desired site; and
   bringing the nanomesh laminate into contact with an aqueous alcohol solution or water.
[9] A nanomesh-attaching kit comprising:
   the nanomesh laminate according to any one of [1] to [7]; and
   a carrier carrying an aqueous alcohol solution or water.

### EFFECT OF THE INVENTION

The present invention can provide a nanomesh laminate which allows simple attachment of a nanomesh material, preferably a conductive nanomesh material, to a desired site, and which is less likely to cause distortion upon the attachment.

Further, by attaching the nanomesh laminate using water or an aqueous alcohol solution after the placement of the nanomesh laminate on the desired site, the time required for the attachment can be reduced, and movement of the conductive substance layer from the desired position can be prevented.

Further, a nanomesh-attaching kit comprising the combination of a nanomesh laminate and a carrier such as absorbent cotton impregnated with water or an aqueous alcohol solution can be provided.

### BRIEF DESCRIPTION OF THE DRAWINGS

Fig. 1 is a schematic cross-sectional view illustrating a conventional embodiment.
Fig. 2 is a schematic cross-sectional view illustrating an embodiment of the present invention.
Fig. 3 is a schematic cross-sectional view illustrating another embodiment of the present invention.
Fig. 4 is a schematic cross-sectional view illustrating another embodiment of the present invention.
Fig. 5 is a schematic cross-sectional view illustrating another embodiment of the present invention.

### MODE FOR CARRYING OUT THE INVENTION

The present invention is described below in detail. The following descriptions on constituent features are examples (representative examples) of embodiments of the present invention, and the present invention is not limited to these contents. The present invention may be carried out with various modifications within the scope of its spirit.

One embodiment of the present invention is a nanomesh laminate comprising:
a mesh-shaped base material (A); and
a nanomesh layer (B) containing a polyvinyl alcohol resin as a main component,
   the mesh-shaped base material (A) and the nanomesh layer (B) being layered next to each other.

The mesh-shaped base material (A) is a base material having a mesh that allows a liquid or vapor to pass through it, and its opening area is not limited. From the viewpoint of increasing permeability to water, an aqueous alcohol solution, water vapor, alcohol vapor, or the like to enable quick attachment, the opening area is preferably 55% to 80%.

On the other hand, when the pattern transferred by the nanomesh is required to be accurate, for example, in cases where the resistance value is measured, or in cases where the transferred pattern has a thin portion, such as cases where part of the transferred pattern has a portion having a thickness of not more than 2 mm, especially a width of only not more than 1 mm, the opening area of the mesh of the base body is preferably 10% to 45%, more preferably not more than 40%, still more preferably not more than 35% for the accurate transfer of the pattern since, in this case, the pressure applied to the nanomesh laminate, especially to the conductive substance layer, upon the attachment can be dispersed, and hence damage on the nanomesh laminate, especially on the conductive substance layer can be suppressed.

In cases where the opening area is low, passage of a gas such as water vapor or alcohol vapor through the mesh-shaped base material may occur more often than passage of a liquid therethrough. This may result in increased condensation on the attachment surface, and hence the nanomesh layer is thought to be more likely to dissolve from the attachment-surface side, thereby enabling the accurate transfer.

The mesh-shaped shape may be a lattice shape, and examples of the mesh-shaped lattice shape include 60 deg. staggered, 45 deg. staggered, straight, round end slot staggered, round end slot straight, square staggered, square straight, hexagonal 60 deg. staggered, square end slot staggered, square end slot straight. Square straight is preferred from the viewpoint of adhesion to the nanomesh layer (B).

In cases where the aperture of the base material has a regular pattern, the opening area may be calculated based on the pattern.

In cases where the aperture of the base material has a regular pattern like a window screen, and also in cases where the aperture has an irregular pattern like a non-woven fabric, the opening area can be determined by binarization of the pattern of the aperture as follows. For example, the pattern may be captured as binary data under a light microscope at a magnification of ×10, and the ratio of the aperture in the total area may be calculated to determine the opening area. Regarding the size of each grid for the binarization, the measurement may be carried out for an area of about 0.1 mm × 0.1 mm. In particular, in cases where the aperture has an irregular pattern, an image may be captured for an area of 1 cm × 1 cm at a magnification of ×10, and the size of each grid may be set to 0.1 mm × 0.1 mm. By determining the ratio of the aperture, the opening area can be obtained.

Regarding the method of confirmation of the opening area, the mesh-shaped base material may be peeled off from the nanomesh laminate, and the nanomesh layer may be removed with a solvent such as water. By observing the mesh-shaped base material using a light microscope, the opening area can be determined as described above.

Examples of the material of the mesh-shaped base material (A) include commonly used thermoplastic resins such as polyethylene, polypropylene, polyamide, polystyrene, and polyester. From the viewpoint of ease of detachment of the nanomesh layer, the material is preferably polyethylene or polypropylene, which has high hydrophobicity. From the viewpoint of flexibility, the material is preferably polyethylene. From the viewpoint of strength, the material is preferably polypropylene.

The thickness of the mesh-shaped base material (A) is usually not less than 10 µm, preferably not less than 50 µm, more preferably not less than 100 µm, and is usually not more than 500 µm, preferably not more than 400 µm, more preferably not more than 300 µm. In cases where the thickness of the mesh-shaped base material (A) is within this range, the nanomesh layer (B) can be sufficiently supported, and the base material can easily follow the adherend. The roughness of the mesh of the mesh-shaped base material (A) is not limited as long as a liquid can permeate. The mesh size is usually not less than 50 nm, preferably not less than 100 nm, and is usually not more than 20 mm, preferably not more than 10 mm.

The nanomesh layer (B) comprises a polyvinyl alcohol (PVA) resin as a main component. In the present description, the "main component" means the component contained in the largest amount, and the amount of the main component may be not less than 50% by weight, may be not less than 70% by weight, may be not less than 90% by weight, or may be not less than 100% by weight in the nanomesh layer (B). Thus, the nanomesh layer (B) may contain a fiber other than the PVA resin. The fiber other than the PVA resin is preferably a water-soluble resin, and examples thereof include synthetic polymers such as polyethylene glycol, polyethylene oxide, polyacrylic acid, polymethacrylic acid, polyvinyl pyrrolidone, and polyacrylamide; natural polymers such as gelatin, pullulan, and water-soluble collagen; and their chemically modified polymers (excluding polysaccharide thickeners).

The PVA resin constituting the nanomesh layer (B) has a fiber diameter of preferably not less than 50 nm, more preferably not less than 100 nm, still more preferably not less than 200 nm, especially preferably not less than 300 nm. The fiber diameter is usually not more than 1000 nm, preferably not more than 900 nm, more preferably not more than 800 nm, still more preferably not more than 600 nm, especially preferably not more than 500 nm. With such a fiber diameter, sufficient durability of the laminate can be achieved, and sufficient density of the conductive substance layer can be easily achieved, which is preferred.

In the electrospinning method, the fiber diameter can be adjusted with, for example, the concentration of the spinning solution used for the production of the nanomesh layer. As the concentration of the PVA resin, or the PVA resin and the water-soluble resin, in the spinning solution increases, the fiber diameter increases. In the melt blowing method, the fiber diameter can be adjusted with, for example, the discharge rate of the spinning solution used in the production of the nanomesh layer. As the discharge rate of the spinning solution composed of the PVA resin, or of the PVA resin and the water-soluble resin, increases, the fiber diameter increases.

Examples of the PVA resin include an unmodified PVA, a carboxyl group-containing PVA, a sulfo group-containing PVA, an acetoacetyl group-containing PVA, and a modified PVA containing a 1,2-diol structure or the like in a side chain. From the viewpoint of biocompatibility, an unmodified PVA is especially preferred.

A PVA resin is a resin obtained by saponification of a vinyl ester resin obtained by polymerization of vinyl ester monomers, and mainly composed of vinyl alcohol structural units. It is composed of vinyl alcohol structural units equivalent to a saponification degree, and vinyl ester structural units that are left unsaponified.

An unmodified PVA is composed only of vinyl alcohol structural units and vinyl ester structural units, and a modified PVA is a PVA containing not only vinyl alcohol structural units and vinyl ester structural units, but also other structural units.

The average polymerization degree of the PVA resin used in the present embodiment is preferably not less than 300, more preferably not less than 300, still more preferably not less than 400, especially preferably not less than 500. Within this range, the strength of the nanomesh can be improved. On the other hand, the average polymerization degree of the PVA resin is preferably not more than 3500, more preferably not more than 3000, still more preferably not more than 2500, especially preferably not more than 2300. Within this range, the PVA resin can be easily produced.

The average polymerization degree of the PVA resin in the present embodiment is the average polymerization degree determined by a method in accordance with JIS K 6726.

The saponification degree of the PVA resin is preferably 70 to 100 mol%, more preferably 70 to 95 mol%, still more preferably 70 to 92 mol%, especially preferably 70 to 90 mol%.

In cases where the saponification degree of the PVA resin is not less than 70 mol%, flexibility and viscosity can be maintained within optimal ranges, so that a molded product can be easily handled. On the other hand, although the PVA resin may be a completely saponified product (that is, a product with a saponification degree of 100 mol%), the production of the nanomesh layer (B) can be more easily achieved when the saponification degree is within the above-described range.

The saponification degree of the PVA resin in the present embodiment is a value determined by a method in accordance with JIS K 6726.

Other examples of the components constituting the nanomesh layer (B) include plasticizers and thickeners.

Examples of the plasticizers that may be added include polyols such as glycerin, sorbitol, and polyethylene glycol; and alkylene oxide adducts thereof. An especially preferred plasticizer for the PVA resin is glycerin. The amount of the plasticizer added is not limited as long as it is sufficient for obtaining a desired flexibility and solubility. The amount is not limited, and usually preferably not more than 30 parts by weight, more preferably 10 to 15 parts by weight with respect to 100 parts by weight of the PVA resin. For a use in which the acceptable degree of film deformation is small, the amount of the plasticizer added is preferably not more than 2 parts by weight with respect to 100 parts by weight of the PVA resin.

Examples of the thickeners that may be used include polysaccharides; galactomannans such as guar gum, tara gum, and locust bean gum; xanthan gum; gellan gums such as low acyl gellan gum (hereinafter also referred to as "LA gellan gum"); fermented polysaccharides such as welan gum, rhamsan gum, and diutan gum; glucoses such as starch and dextrin; cellulose derivatives such as sodium carboxymethyl cellulose, hydroxyethyl cellulose, and hydroxypropyl methyl cellulose; and pullulan. Polysaccharide thickeners other than these may also be used as long as they have thickening properties.

Examples of the method of producing the nanomesh layer (B) include the electrospinning method (electric field spinning method), the melt blowing method, and the sea-island melt spinning method.

The electrospinning method may be carried out by well-known means. Generally, a solution prepared by dissolving a polymer material (spinning solution) is filled into a syringe, and high voltage is applied between the syringe nozzle and a conductive collector to cause jet-like scattering of the solution. The solvent is volatilized in the process of scattering, resulting in accumulation of a fiber in the collector.

For formation of the nanomesh layer (B) on a support, a conductive support may be used as it is as a collector. Alternatively, a support may be placed between the nozzle and the collector.

The melt blowing method is a common method of producing a non-woven fabric. For production of the nanomesh, the nozzle diameter is reduced to reduce the discharge rate of the resin.

In the sea-island melt spinning method, a sea-island composite fiber with tens to hundreds of islands is prepared using a mouthpiece capable of arranging a large number of island component polymer in a sea component polymer, and then the sea component polymer is removed therefrom using a solvent to obtain an ultrafine fiber composed of the island component polymer.

The conditions for carrying out the electrospinning method are not limited, and may be controlled depending on the type of the spinning solution, the intended use of the ultrafine fiber obtained, and the like. For example, the following conditions may be commonly used: applied voltage, 5 to 30 kV; discharge rate, 0.01 to 1.00 mL/minute; vertical distance between the nozzle and the substrate, 100 to 200 mm; diameter of the nozzle, 15 to 25 G. Although the spinning conditions do not necessarily need to be strictly controlled, the relative humidity is preferably 10 to 50 RH%, and the temperature is preferably 10 to 25°C.

The solvent used for the spinning solution to be subjected to the electrospinning method is preferably a good solvent for the PVA resin. In cases where a water-soluble resin is additionally used, the solvent is preferably a good solvent for the PVA resin and the water-soluble resin. In cases where a polysaccharide thickener is also additionally added, the solvent is preferably a good solvent for the PVA resin and the water-soluble resin, but a poor solvent for the polysaccharide thickener. Such a solvent is preferably water or an alcohol. By dissolving the water-soluble resin using such a solvent, and adding the polysaccharide thickener thereto, a solution of the water-soluble resin in which the polysaccharide thickener is dispersed can be obtained. By spinning the obtained dispersion by the electrospinning method, a laminate sheet in which the polysaccharide thickener is present in the form of particles in a fiber of the water-soluble resin can be obtained. For use as a cosmetic or pharmaceutical, it is preferred to use water, ethanol, or ethylene glycol as the solvent from the viewpoint of safety.

The concentration of the PVA resin, or the PVA resin and the water-soluble resin, in the spinning solution may be changed depending on the solvent used, and the type and the average molecular weight of the water-soluble resin. The concentration is usually about 5 to 20% by weight. In cases where the concentration of the PVA resin, or the PVA resin and the water-soluble resin, is too low, scattering of droplets from the tip of the nozzle does not lead to formation of the fiber. In cases where the concentration is too high, the solution discharged from the nozzle does not scatter like a jet before the solution reaches the collector, so that the solution reaches the collector as it is, or solidification of the solution occurs at the tip of the nozzle, preventing discharge of the solution, and the fiber sheet thus cannot be obtained.

The amount of the polysaccharide thickener added to the spinning solution is preferably 10 to 900 parts by weight, more preferably 20 to 600 parts by weight, still more preferably 50 to 400 parts by weight with respect to 100 parts by weight of the water-soluble resin. Depending on the areal weight of the sheet, in cases where the amount added is not more than 10 parts, a sufficient thickening effect cannot be obtained, while in cases where the amount added is not less than 900 parts by weight, the fiber for forming the skeleton of the sheet will be so insufficient that strength wil be decreased and it will be difficult to handle the obtained sheet.

The spinning solution may optionally contain a conductive agent or a surfactant. Their amounts added are usually 0.0001 to 5% by weight with respect to the spinning solution. By the addition of the conductive agent and/or the surfactant, formability of the fiber can be improved. The conductive agent is preferably a salt soluble in the solvent, and examples of the conductive agent include lithium salts, sodium salts, potassium salts, magnesium salts, calcium salts, aluminum salts, and ammonium salts. Examples of the surfactant include anionic, cationic, nonionic, or amphoteric surfactants. From the viewpoint of reducing influence on the spinning, nonionic surfactants are preferred.

As long as the spinning by the electrospinning method is not inhibited, the spinning solution may contain a hydrocarbon such as paraffin wax, squalane, or vaseline; a natural wax such as carnauba wax or beeswax; an ester such as octyldodecyl palmitate, isopropyl myristate, or glyceryl trioctanoate; a fatty acid such as stearic acid or behenic acid; an oil or fat such as a lanolin derivative or an amino acid derivative; a silicone compound such as dimethylpolysiloxane, methylphenylpolysiloxane, alkyl-modified organopolysiloxane, alkoxy-modified organopolysiloxane, higher fatty acid-modified organopolysiloxane, or fluorine-modified organopolysiloxane; a fluorine oil agent such as perfluoropolyether, perfluorodecane, or perfluorooctane; an oil-based gelling agent such as dextrin fatty acid ester, sucrose fatty acid ester, starch fatty acid ester, aluminum isostearate, or calcium stearate; an ultraviolet inhibitor such as a benzophenone-based inhibitor, a PABA-based inhibitor, a cinnamic acid-based inhibitor, a salicylic acid-based inhibitor, titanium oxide, or cerium oxide; a cosmetic component such as collagen peptide, silk fibroin, sodium hyaluronate, sodium salicylate, arbutin, citric acid, magnesium L-ascorbate phosphate, lactoferrin, ascorbyl tetrahexyl decanoate, arbutin, gamma-oryzanol, vitamin A acetate, panthenol, allantoin, or betaine trimethylglycine; a perfume; or the like.

For cosmetic uses, the spinning solution may contain an effective cosmetic component. Examples of the effective cosmetic component include skin-whitening components, ultraviolet-protective components, anti-aging components, anti-wrinkle components, anti-inflammatory components, antioxidant components, moisturizing components, astringent components, slimming components, peeling components, skin-beautifying components, blood circulation-promoting components, antimicrobial components, microbicidal components, and refreshing components. For pharmaceutical uses, the spinning solution may contain a pharmaceutically effective component or a skin protective component such as an anti-inflammatory component, an antihistamine component, an antiallergic component, a blood circulation-promoting component, a vasodilator component, an anti-inflammatory component, an analgesic component, a nutritional component, a wound healing component, an antimicrobial component, an antiviral component, or a microbicidal component. Further, for promoting percutaneous absorption of these effective components, the spinning solution may contain a percutaneous absorption-promoting agent, for example, an alcohol such as menthol, camphor, or cetyl alcohol; a fatty acid ester such as isopropyl palmitate or isopropyl myristate; a glycerin ester such as glycerin monolaurate or glycerin monooleate; an acid amide such as diethanolamide laurate; a neutral surfactant such as polyethylene glycol dilauryl ether; an unsaturated fatty acid such as levulinic acid, capric acid, lauric acid, myristic acid, palmitic acid, stearic acid, isostearic acid, or oleic acid; or an ionic liquid.

For cosmetic or pharmaceutical uses, the content of each effective component is not limited as long as its effect can be exerted. The content is usually 0.0001% by weight or more and 10% by weight or less. In cases where a component to be included in the spinning solution is insoluble in the good solvent for the PVA resin and/or the water-soluble resin, the component to be included may be dissolved in a solvent other than the good solvent for the PVA resin and/or the water-soluble resin. In such cases, the solution in which the component to be included is dissolved may be dispersed, using a homogenizer or stirrer, in the solution in which the PVA resin, or the PVA resin and the water-soluble resin, is/are dissolved, and the resulting emulsion or suspension solution may be used as the spinning solution.

The nanomesh laminate of the present embodiment preferably comprises: a nanomesh layer (B) containing a PVA resin as a main component; and a conductive substance layer (C), on a mesh-shaped base material (A).

In a preferred mode, the nanomesh layer is composed of PVA (polyvinyl alcohol), which is a biodegradable resin, and the conductive material is composed of gold. Therefore, the nanomesh laminate can be preferably applied to a living body, but may be used, when applicable, as a flexible electronic device. In cases of application to a living body, the nanomesh laminate may be used as a sensor for measuring the temperature, pressure, myogenic potential, and/or the like of a living body. A detailed description is given below using drawings.

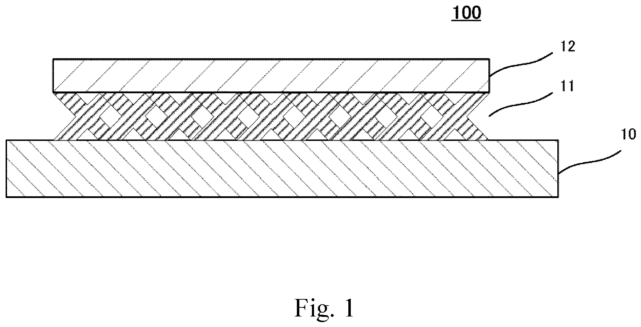

Fig. 1 is a schematic cross-sectional view illustrating a nanomesh sheet as a conventional embodiment.

A nanomesh sheet 100 can be produced by forming a nanomesh 11 on a PET film 10 using the electrospinning method or the like, and then forming a conductive substance layer 12 thereon by the vapor deposition method, the sputtering method, or the like. The nanomesh sheet 100 has the problem that the nanomesh is broken upon detachment from the PET film 10. In order to solve this problem, the present inventors have proposed adjustment of the peel force on the surface of the release material used as a base material (Japanese Patent Application No. 2019-065956).

On the other hand, since the nanomesh after the detachment from the release material was thin and light, it could not be easily attached to a desired site. For example, in cases where the attachment site is small, positioning is difficult, while in cases where the attachment site is large, distortion occurs between the portion that has already adhered and the portion that has not yet adhered, leading to difficulty in achieving uniform attachment. In order to solve such a problem, a mesh-shaped base material (A) is used in the present embodiment.

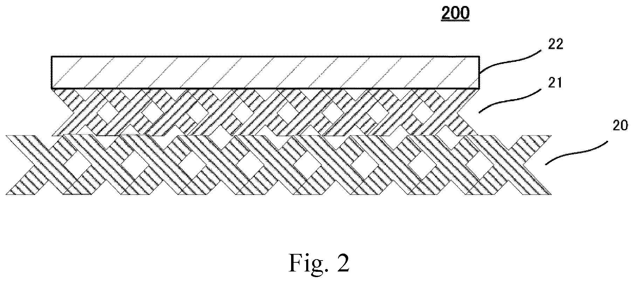

Fig. 2 is a schematic cross-sectional view illustrating one example of a nanomesh laminate according to the present embodiment.

In a nanomesh sheet 200, a PVA nanomesh 21 and a conductive substance layer 22 are layered on a mesh-shaped base material 20.

The mesh-shaped base material 20 supports the laminate of the PVA nanomesh sheet 21 and the conductive substance layer 22, which are layered. As described above, the mesh-shaped base material is not limited as long as it is hardly soluble and allows a liquid or vapor to pass through the mesh.

The mesh-shaped base material 20 may be a commercially available product such as a porous film, or may be produced by a known method.

The PVA nanomesh layer 21 is a nanomesh layer containing a PVA resin as a main component. Preferably, it is composed of a PVA resin, and can be dissolved with water or alcohol. The PVA nanomesh layer 21 is typically prepared by the electrospinning method. In the electrospinning method, the mesh-shaped base material is provided, and the PVA nanomesh layer is formed thereon. The electrospinning method is a method used also in Patent Document 1, and one may refer to the document.

In the present embodiment, the PVA nanomesh layer 21 may be substituted with a biodegradable resin other than a PVA resin. For example, polyvinyl pyrrolidone (PVP), polyglycolic acid (PGA), polylactic acid (PLA), or the like may be used.

On the PVA nanomesh layer 21, the conductive substance layer 22 is formed. The conductive material is not limited as long as it has conductivity, and specific examples of the conductive material include metals such as gold, platinum, silver, silver chloride, copper, titanium, palladium, chromium and cobalt, and alloys thereof; and carbon. In cases where a transparent conductive material is desired, ITO (indium tin oxide) may be used. A conductive metal oxide other than these, such as nickel oxide, tin oxide, indium oxide, indium-zirconium oxide (IZO), titanium oxide, or zinc oxide may also be used. PEDOT: PSS, which is a polythiophene derivative doped with polystyrene sulfonate, PEDOT: PTS, which is a polythiophene derivative doped with paratoluene sulfonate, or a conductive polymer material prepared by doping of polypyrrole, polyaniline, or the like with iodine or the like, may also be used. Among these, taking application to a living body into account, gold, carbon, titanium, PEDOT: PSS, or PEDOT: PTS is preferred. Gold is especially preferred.

The method of forming the conductive substance layer is not limited, and it is formed by a dry process or an application method. Examples of the dry process include vapor deposition, sputtering, and CVD. Among these, the conductive substance layer can be most simply formed by vapor deposition. Specific examples of the application method include, but are not limited, the wire bar coating method, blade coating method, die coating method, slit die coating method, reverse roll coating method, gravure coating method, kiss coating method, roll brush method, spray coating method, air knife coating method, pipe doctor method, impregnation-coating method, curtain coating method, flexography method, screen printing method, and ink-jet method.

The thickness of the PVA nanomesh layer 21 is not limited, and may be appropriately set depending on the purpose. The thickness is usually not less than 0.1 µm, may be not less than 1 µm, and is usually not more than 100 µm, may be not more than 80 µm.

The thickness of the conductive substance layer is not limited. It is usually not less than 5 nm, may be not less than 10 nm, and is usually not more than 2 µm, may be not more than 1 µm, or may be not more than 500 nm.

By attaching the nanomesh laminate of the present embodiment to a subject such as the skin such that the mesh-shaped base material is positioned in the upper side, and bringing water or alcohol into contact with the PVA nanomesh, the PVA nanomesh can be dissolved. As the liquid dries, the PVA acts as an adhesive to allow formation of a conductive substance layer, that is, wiring or a conductive circuit on the subject.

In this process, water may be used. However, use of an aqueous alcohol solution rather than water improves the drying rate of the liquid, so that curling of the PVA nanomesh is less likely to occur, and slipping is suppressed, which is preferred.

The alcohol is preferably an alcohol capable of accelerating evaporation of a liquid containing water. The alcohol preferably contains ethyl alcohol or methyl alcohol, or isopropyl alcohol. A mixture of these may also be used. The mixing ratio to water may be selected within the range of 5:95 to 98:2. In particular, the ratio of ethanol is preferably 90% to 50%, and an alcohol for use in disinfection (70% to 90% ethyl alcohol) is especially preferred. A surfactant or the like may also be included.

On the other hand, in cases where a nanomesh laminate containing an effective cosmetic component or a pharmaceutically effective component is attached to the skin, alcohol-free water is preferably used from the viewpoint of safety. In such cases, a surfactant or the like may also be included.

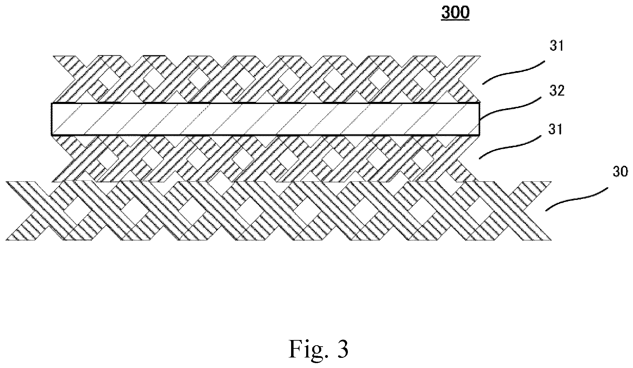

Another embodiment of the present invention is illustrated in Fig. 3.

A nanomesh sheet 300 of the present embodiment is a mode in which a conductive substance layer 32 is sandwiched from both sides with a PVA nanomesh 31. In cases where the conductive substance layer 32 has a large area, when the PVA nanomesh 31 is present only on one side, the adhesive force to the subject may be insufficient. By providing the PVA nanomesh 31 on both sides of the conductive substance layer 32 as in the present embodiment, the conductive substance layer 32 can be allowed to adhere to the subject securely.

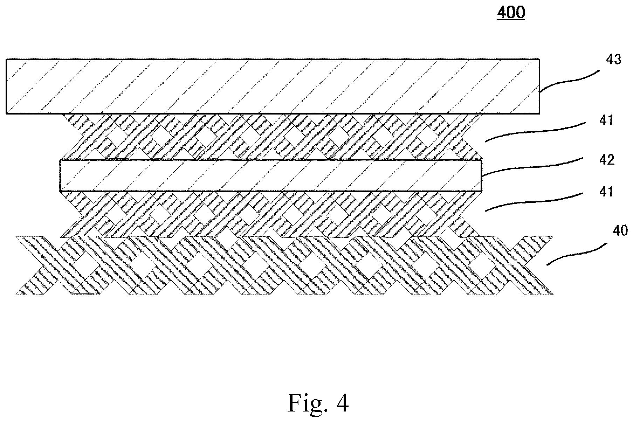

Still another embodiment of the present invention is illustrated in Fig. 4.

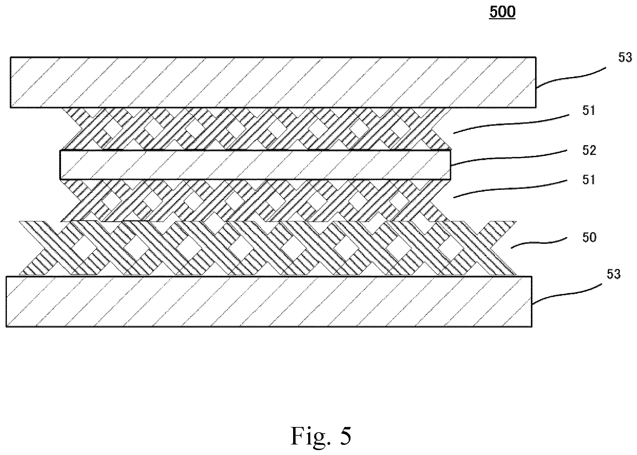

The nanomesh sheet 400 of the present embodiment has a protection layer 43. Since the PVA nanomesh 41 is soluble in water, it is sensitive to humidity. Thus, it is preferred to provide the protection layer 43 for protecting the PVA nanomesh 41 against moisture. Further, as illustrated in Fig. 5, protection layers may be provided on both sides of the PVA nanomesh 51.

The protection layer is not limited as long as the PVA nanomesh layer and the conductive substance layer can be protected against moisture and the like. The protection layer preferably has gas barrier properties. More specifically, the protection layer is preferably a resin film of PET, PTEF, EVA, or the like.

The thickness of the protection layer is not limited. It may be not less than 0.5 µm, or may be not less than 1 µm, and may be not more than 2000 µm, or may be not more than 1000 µm.

The embodiments of the present invention also include a method of producing a conductive circuit using a nanomesh laminate described above. More specifically, another embodiment of the present invention is a method of producing a conductive circuit, the method comprising the steps of: placing the nanomesh laminate on a desired site; and bringing the nanomesh laminate into contact with an aqueous alcohol solution or water. The site where the conductive circuit is formed is not limited. It is preferably applied to the skin of a human body.

Still another embodiment is a nanomesh-attaching kit comprising: the nanomesh laminate; and a carrier carrying an aqueous alcohol solution or water. By providing the nanomesh-attaching kit, a conductive circuit can be easily formed on a human body. Therefore, a sensor for measuring various parameters of a diseased patient can be very easily attached. Moreover, the same circuit can be stably provided for many patients. Moreover, since the circuits formed in each patient exhibit only small variation, failure in formation of the circuit can be reduced.

The carrier that carries the aqueous alcohol solution or water is not limited as long as it is capable of carrying the aqueous alcohol solution or water. Examples of the carrier include cotton, nylon, woven polyester fabric, and non-woven polyester fabric. As described above, the aqueous alcohol solution is preferably capable of accelerating evaporation of a liquid containing water. It preferably contains ethyl alcohol or methyl alcohol, or isopropyl alcohol. A mixture of these may also be used. The mixing ratio to water may be selected within the range of 5:95 to 98:2. In particular, the ratio of ethanol is preferably 90% to 50%, and an alcohol for use in disinfection (around 80% ethyl alcohol) is especially preferred. The aqueous alcohol solution or water may also contain a surfactant or the like.

### EXAMPLES

The present invention is described below in more detail by way of Examples. Needless to say, however, the scope of the present invention is not limited to the descriptions in the Examples.

### (Example 1)

### [Providing of Mesh-shaped Base Material (A)]

A mesh-shaped base material (A) (opening area, 65.6%; square straight mesh; made of polyethylene; thickness, 240 µm) was loaded in the nanomesh-forming section (on the drum) of a drum-type electrospinning apparatus manufactured by Kato Tech Co., Ltd.

### [Production of Nanomesh Layer (B)]

PVA (unmodified; saponification degree, 88 mol%; 4% by weight aqueous solution; viscosity, 3 mPa·s) was dissolved in ethylene glycol to prepare a 4% by weight PVA solution in ethylene glycol, to provide a spinning solution.

In the electrospinning apparatus on which the mesh-shaped base material (A) provided as described above was placed, 4 ml of the spinning solution was fed into a syringe, and an electrode was attached to an 18 G non-bevel needle (manufactured by Terumo Corporation) at the tip of the syringe. A nanomesh layer (B) was then produced by electrospinning, to obtain a nanomesh laminate 1. The following settings were used for the electrospinning apparatus.

In the present Example, a nanomesh layer (B) without formation of a circuit was used instead of a sensor layer in which a circuit is formed with a conductive substance. The presence or absence of circuit formation does not affect handling properties.

### <Settings>

Target speed: 5 m/min
Traverse speed: 10 cm/min
Syringe speed: 0.050 to 0.080 mm/min
Voltage: 15 to 20 kV
Film formation time: 60 min

### [Evaluation of Handling Properties]

The nanomesh laminate 1 obtained as described above was placed on the skin of a subject whose body temperature was about 36°C, such that the conductive-material side was in contact with the skin. Water was sprayed onto the mesh-shaped base material (A) in an indoor environment at about 23°C. The mesh-shaped base material (A) could be easily removed, and a state where the nanomesh layer (B) was adhering to the skin without distortion could be obtained.

### (Reference Example 1)

According to a conventional method, a nanomesh laminate was prepared in the same manner as in Example 1 except that a silicone-processed greaseproof paper (cooking sheet 0251; 30 cm × 5 m; manufactured by Toyo Aluminium K. K.) was used as the base material. The nanomesh laminate was then subjected to evaluation of handling properties. The laminate obtained could be easily removed from the base material without breakage. However, the nanomesh curled upon the attachment to the skin, and adhered to itself, so that the laminate could not be attached to the skin without wrinkles.

### (Comparative Example 1)

A PVA nanomesh sheet having the conventional configuration illustrated in Fig. 1 was used. The size of the nanomesh sheet was about 3 × 5 cm, and three gold wires of 0.5 cm × 4 cm were provided for mimicking a circuit. The base material was detached from the nanomesh sheet, and the nanomesh sheet was placed on the skin of a subject whose body temperature was about 36°C. In an indoor environment at about 23°C, medical alcohol cotton (absorbent cotton impregnated with 80% ethyl alcohol and 20% water) was pressed onto the nanomesh sheet for not less than 1 minute. As a result, the gold wires were transferred to the alcohol-cotton side, and a circuit could not be formed well on the skin.

### (Comparative Example 2)

Instead of the alcohol cotton, a sprayer was used to spray water on the PVA nanomesh sheet. As a result, the gold wires deformed, and the desired circuit drawn in the mesh-sheet shape could not be transferred.

### (Example 2)

The PVA nanomesh laminate using a mesh-shaped base material (A) (made of polypropylene), illustrated in Fig. 2 was used. The nanomesh laminate had a size of about 3 × 5 cm, and the mesh size of the mesh-shaped base material (A) was 1000 µm. The diameter of the fiber constituting the mesh of the mesh-shaped base material (A), having an opening area of 62.3%, was 0.7 µm. Three gold wires of 0.4 cm × 3 cm were provided for mimicking a circuit. The nanomesh laminate, including the mesh-shaped base material (A), was placed on the skin of a subject whose body temperature was about 36°C. In an indoor environment at about 23°C, medical alcohol cotton (alcohol cotton impregnated with 80% ethyl alcohol and 20% water) was pressed onto the mesh-shaped base material (A) of the nanomesh sheet for not less than 1 minute. As a result, the gold wires could be transferred to the skin while maintaining their shapes, thereby successfully forming a circuit.

### (Example 3)

The PVA nanomesh laminate sheet using a mesh-shaped base material (A) (made of polypropylene; opening area, 26%; square straight mesh), illustrated in Fig. 2 was used. The size of the nanomesh laminate sheet was about 3 × 5 cm, and three gold wires of 0.4 cm × 3 cm were provided for mimicking a circuit. The nanomesh laminate sheet, including the mesh-shaped base material (A), was placed on the skin of a subject whose body temperature was about 36°C. In an indoor environment at about 23°C, absorbent cotton impregnated with only water was pressed onto the mesh-shaped base material (A) of the nanomesh sheet for not less than 1 minute. As a result, the gold wires could be transferred to the skin while maintaining their shapes, thereby successfully forming a circuit.

### (Example 4)

The PVA nanomesh laminate sheet using a mesh-shaped base material (A) (made of polypropylene; opening area, 26%; square straight mesh), illustrated in Fig. 2 was used. The size of the nanomesh laminate sheet was about 3 × 5 cm, and three gold wires of 0.4 cm × 3 cm were provided for mimicking a circuit. The nanomesh laminate sheet, including the mesh-shaped base material (A), was placed on the skin of a subject whose body temperature was about 36°C. In an indoor environment at about 23°C, medical alcohol cotton (alcohol cotton impregnated with 80% ethyl alcohol and 20% water) was pressed onto the mesh-shaped base material (A) of the nanomesh sheet for not less than 1 minute. As a result, the gold wires could be transferred to the skin while maintaining their shapes, thereby successfully forming a circuit.

### DESCRIPTION OF SYMBOLS

100, 200, 300, 400, 500 Nanomesh sheet
10 PET film
20, 30, 40, 50 Mesh-shaped base material (A)
11, 21, 31, 41, 51 PVA nanomesh
12, 22, 32, 42, 52 Conductive substance
43, 53 Protection film

## Claims

1. A nanomesh laminate comprising:
a mesh-shaped base material (A); and
a nanomesh layer (B) containing a polyvinyl alcohol resin as a main component,
the mesh-shaped base material (A) and the nanomesh layer (B) being layered next to each other.

2. The nanomesh laminate according to claim 1, comprising:
the nanomesh layer (B) containing a polyvinyl alcohol resin as a main component; and
a conductive substance layer (C),
on the mesh-shaped base material (A).

3. The nanomesh laminate according to claim 1 or 2, wherein
the nanomesh layer (B) containing a polyvinyl alcohol resin as a main component,
the conductive substance layer (C), and
a protection layer for protecting these,
are layered in this order on the mesh-shaped base material (A) .

4. The nanomesh laminate according to any one of claims 1 to 3, wherein the mesh-shaped base material (A) has an opening area of 55 to 80%.

5. The nanomesh laminate according to any one of claims 1 to 3, wherein
a mesh-shaped base material (A) having an opening area of 55 to 80%, and
a nanomesh layer (B1) containing a polyvinyl alcohol resin as a main component, and containing a cosmetic component or a pharmaceutical component,
are layered next to each other.

6. The nanomesh laminate according to any one of claims 1 to 3, wherein the mesh-shaped base material (A) has an opening area of 10 to 45%.

7. The nanomesh laminate according to any one of claims 1 to 3, wherein
a mesh-shaped base material (A) having an opening area of 10 to 45%, and
a nanomesh layer (B1) containing polyvinyl alcohol as a main component and containing a cosmetic component or a pharmaceutical component,
are layered next to each other.

8. A method of producing a conductive circuit, comprising the steps of:
placing the nanomesh laminate according to any one of claims 1 to 7 on a desired site; and
bringing the nanomesh laminate into contact with an aqueous alcohol solution or water.

9. A nanomesh-attaching kit comprising:
the nanomesh laminate according to any one of claims 1 to 7; and
a carrier carrying an aqueous alcohol solution or water.
